# EUROPÄISCHE PATENTSCHRIFT

(11) **EP 4 034 342 B1**
(45) Veröffentlichungstag und Bekanntmachung des Hinweises auf die Patenterteilung: **09.08.2023**
(21) Anmeldenummer: 20771318.1
(22) Anmeldetag: 17.09.2020
(51) Int. Cl.: B25H 1/00, F16M 13/04

(54) **VORRICHTUNG ZUR BEREITSTELLUNG EINER UNTERSTÜTZUNGSKRAFT FÜR DIE OBEREN EXTREMITÄTEN EINES NUTZERS SOWIE EIN EXOSKELETT**
DEVICE FOR PROVIDING AN ASSISTING FORCE FOR UPPER EXTREMITIES OF A USER AND AN EXOSKELETON
DISPOSITIF DE FOURNITURE D'UNE FORCE DE SUPPORT AUX EXTRÉMITÉS SUPÉRIEURES D'UN UTILISATEUR AINSI QU'EXOSQUELETTE

(30) Priorität: 23.09.2019 EP 19198859
(43) Veröffentlichungstag der Anmeldung: 03.08.2022
(73) Patentinhaber: Hilti Aktiengesellschaft, 9494 Schaan (LI)
(72) Erfinder: KÄSMANN, Simon, 81241 München (DE)
(74) Vertreter: Hilti Aktiengesellschaft Corporate Intellectual Property
(86) Internationale Anmeldenummer: PCT/EP2020/075965
(87) Internationale Veröffentlichungsnummer: WO 2021/058366

(56) Entgegenhaltungen:
- US-A- 5 360 196
- US-A1- 2018 111 262
- US-A1- 2018 364 004

## Beschreibung

Die vorliegende Erfindung betrifft eine Vorrichtung zur Bereitstellung einer Unterstützungskraft für die oberen Extremitäten eines Nutzers, sowie ein Exoskelett, das mindestens eine solche Vorrichtung umfasst. Der wesentliche Vorteil der Erfindung besteht darin, dass mit der vorgeschlagenen Vorrichtung eine Unterstützungskraft für die oberen Extremitäten eines Nutzers in zwei unterschiedlichen Kraftbereichen bereitgestellt werden kann. Dadurch kann das vorgeschlagene Exoskelett in zwei verschiedenen Betriebszuständen oder -modi betrieben werden und weist ein besonders breites Leistungsspektrum im Vergleich zu anderen Exoskeletten auf. Vorzugsweise werden die beiden Betriebszustände durch die Vorsehung eines Federelements ermöglicht, das mit einem Verbindungselement und einem Kraftübertragungselement zusammenwirkt. Es stellt eine Abkehr vom Stand der Technik dar, dass zwei unterschiedliche Kraftmodi bereitgestellt werden können, ohne dass dafür zwei Federelemente, die insbesondere unterschiedliche Federhärten aufweisen können, verwendet werden müssen. Dadurch kann der Aufbau der Vorrichtung bzw. des Exoskeletts bei gleicher Funktionalität gegenüber konventionellen Vorrichtungen, die aus dem Stand der Technik bekannt sind, wesentlich vereinfacht werden, so dass mit der Erfindung besonders robuste, insbesondere baustellentaugliche Vorrichtungen bzw. Exoskelette bereitgestellt werden können, die beispielsweise im Baugewerbe Anwendung finden können.

### Hintergrund der Erfindung:

Im modernen Arbeitsleben gibt es körperlich belastende Tätigkeiten, die ein Gesundheitsrisiko für den betroffenen Arbeiter darstellen können. Zu diesen körperlich belastenden Tätigkeiten gehört insbesondere die Arbeit über Kopf, bei der der Arbeiter Tätigkeiten oberhalb des Schulterniveaus ausführt. Dabei kann es sich zum Beispiel um die Montage von komplexen Industrieprodukten, wie Kraftfahrzeugen, handeln. Es können aber beispielsweise auch handwerkliche Tätigkeiten darunterfallen, wie zum Beispiel das Bearbeiten von Decken und hochgelegenen Wänden im Baubereich oder auf Baustellen. Diese Überkopfarbeiten stellen besonders deshalb ein Gesundheitsrisiko für den betroffenen Arbeiter dar, weil die Arbeiten und das Halten der Arme über dem Kopf entgegen der Gravitation erfolgt, wobei die Gravitation in bekannter Weise eine nach unten, d.h. eine in Richtung des Erdmittelpunktes gerichtete Kraft darstellt. Insofern sind die Belastungen für die Arbeiter besonders dann gegeben, wenn schwere Lasten über einen längeren Zeitraum hinweg über dem Kopf oder den Schultern des Arbeiters gehalten werden müssen, wie dies beispielsweise beim Durchführen von Arbeiten mit Elektrowerkzeugen im Baugewerbe der Fall ist.

Um die Belastungen für die Arbeiter zu reduzieren, sind im Stand der Technik sogenannte Exoskelette vorgeschlagen worden, mit denen die Auswirkung der Gravitationskraft zumindest teilweise abgemildert werden kann. Diese Abmilderung erfolgt bei konventionellen Exoskeletten mit Unterstützungskräften, die der Gravitation entgegenwirken, d.h. in eine Raumrichtung «nach oben». Dadurch werden zum Beispiel die oberen Extremitäten des Arbeiters nach oben gedrückt oder es wird verhindert, dass die oberen Extremitäten des Nutzers des Exoskeletts unter ein zuvor festgelegtes Höhenniveau fallen. Beispielsweise kann das Ablegen der oberen Extremitäten auf einer Armstrebe ermöglicht werden, die beispielsweise auf Höhe der Schulter des Nutzers in einer Ebene arretiert ist, wobei die Arretierungsebene im Wesentlichen orthogonal zu einer gedanklichen Achse ausgebildet ist, die im Wesentlichen parallel zur Wirbelsäule des Nutzers verläuft. Ferner sind im Stand der Technik Exoskelette bekannt, die beispielsweise in zwei Betriebszuständen betrieben werden können. Im Stand der Technik wird zum Beispiel vorgeschlagen, dass in den unterschiedlichen Betriebszuständen unterschiedliche große Unterstützungskräfte bereitgestellt werden können. Dazu schlägt der Stand der Technik vor, mehrere Federelemente zu kombinieren, die je nach Schaltung unterschiedliche resultierende Steifigkeiten aufweisen. Diese Konstruktionen mit mehreren Federelementen haben sich aber als sehr schmutz- oder staubanfällig herausgestellt, so dass sie nur schwer beispielsweise auf einer Baustelle eingesetzt werden können. Darüber hinaus erschwert der durch die zwei Federn bedingte aufwändige Aufbau eine Verwendung der bekannten Exoskelette im Baugewerbe weiter.

Beispielsweise offenbart die US 2018 111 262 A1, die die Präambel des Hauptanspruches offenbart, ein armstützendes Exoskelett mit einer Schulterbasis, die mit einem Armverbindungsmechanismus gekoppelt ist. Das Exoskelett umfasst darüber hinaus einen Zugkraftgenerator, der ein Drehmoment bereitstellen kann, sowie einen Vorsprung, der sich im Wesentlichen an dem Drehgelenk befindet. Der Vorsprung kann den Zugkraftgenerator beschränken, wenn sich ein distales Verbindungsglied des Exoskeletts über einen Kippwinkel hinaus erstreckt. Wenn der Vorsprung den Zugkraftgenerator nicht einschränkt, neigt das Drehmoment dazu, das distale Verbindungsglied relativ zu einem proximalen Verbindungsglied zu biegen, wodurch die zum Anheben des Arms erforderlichen menschlichen Schulterkräfte und Drehmomente verringert wird.

Aufgabe der vorliegenden Erfindung ist es somit, die vorstehend beschriebenen Nachteile konventioneller Unterstützungsvorrichtungen zu überwinden und eine Vorrichtung zur Bereitstellung einer Unterstützungskraft für die oberen Extremitäten eines Nutzers, sowie eine Exoskelett bereitzustellen, die einen einfachen Aufbau aufweisen und besonders robust gegenüber Staub und Verschmutzungen sind, so dass sie insbesondere auf Baustellen, im Handwerk bzw. im Baugewerbe verwendet werden können. Darüber hinaus sollen die bereitzustellende Vorrichtung und das bereitzustellende Exoskelett besonders leicht ausgebildet sein, so dass sie keine zusätzliche Gewichtsbelastung für den Arbeiter darstellen.

Die Aufgabe wird gelöst durch den Gegenstand der unabhängigen Ansprüche. Vorteilhafte Ausführungsformen zu dem Gegenstand der unabhängigen Ansprüche finden sich in den abhängigen Ansprüchen. Insbesondere wird die Aufgabe gelöst durch eine Unterstützungsvorrichtung, die in bevorzugt zwei verschiedenen Betriebszuständen betrieben werden kann.

### Beschreibung der Erfindung:

Die Aufgabe wird gelöst durch eine Vorrichtung zur Bereitstellung einer Unterstützungskraft für die oberen Extremitäten eines Nutzers, wobei die Unterstützungskraft in einem ersten Modus und in einem zweiten Modus bereitgestellt wird und wobei die Vorrichtung eine Stützvorrichtung und eine Armstrebe umfasst, die über ein Drehgelenk derart miteinander verbunden vorliegen, dass die Armstrebe in Bezug auf die Stützvorrichtung um eine Rotationsachse drehbar gelagert ist. Die Vorrichtung ist dadurch gekennzeichnet, dass sie ein Federelement zur Bereitstellung der Unterstützungskraft umfasst, wobei das Federelement zwischen der Stützstruktur und einem Verbindungselement angeordnet vorliegt, wobei die Vorrichtung ein Kraftübertragungselement umfasst, das ein erstes Ende und ein zweites Ende aufweist und das um das Verbindungselement führbar ist, wobei die Enden des Kraftübertragungselements an einem ersten Aufhängepunkt an der Stützvorrichtung und einem zweiten Aufhängepunkt an der Armstrebe anbringbar sind, wobei im ersten Modus das erste Ende des Kraftübertragungselements am ersten Aufhängepunkt und ein zweites Ende des Kraftübertragungselements am zweiten Aufhängepunkt angebracht vorliegt und im zweiten Modus das erste und das zweite Ende des Kraftübertragungselements am zweiten Aufhängepunkt angebracht vorliegen.

Es hat sich gezeigt, dass mit der Erfindung die Funktionalität, dass zwei unterschiedliche Betriebs- oder Unterstützungsmodi eingestellt werden können, überraschenderweise auch durch die erfindungsgemäß vorgeschlagene Verwendung eines Federelements, das mit einem Verbindungselement und einem Kraftübertragungselement zusammenwirkt, bereitgestellt werden kann. Es stellt eine Abkehr vom Stand der Technik dar, dass zwei unterschiedliche Kraftmodi bereitgestellt werden können, ohne dass dafür zwei oder mehr Federelemente verwendet werden müssen. Die Fachwelt war bisher davon ausgegangen, dass eine solche Funktionalität insbesondere mit zwei Federn unterschiedlicher Steifigkeit bereitgestellt werden kann. Dies führt aber üblicherweise zu einem aufwändigen Aufbau des Exoskeletts mit vielen einzelnen Bestandteilen. Durch die Erfindung kann nun der Aufbau der vorgeschlagenen Vorrichtung bzw. des vorgeschlagenen Exoskeletts bei gleicher Funktionalität gegenüber konventionellen Vorrichtungen, die aus dem Stand der Technik bekannt sind, wesentlich vereinfacht werden, so dass mit der Erfindung besonders robuste, insbesondere baustellentaugliche Vorrichtungen bzw. Exoskelette bereitgestellt werden können, die besonders geeignet sind für einen Einsatz im Freien, beispielsweise auf Baustellen. Insbesondere hat sich gezeigt, dass die erfindungsgemäßen Vorrichtungen und Exoskelette besonders wenig anfällig gegenüber dem Eindringen von Staub und Feuchtigkeit sind.

Anwendungstests haben ferner gezeigt, dass durch die vorgeschlagene Konstruktion unter Verwendung des Federelements, des Verbindungselements und des Kraftübertragungselements eine besonders leichte Vorrichtung bzw. ein besonders leichtes Exoskelett bereitgestellt werden kann. Dies wird insbesondere durch die Vorsehung des einen Federelements erreicht, dessen Gewicht geringer ist als das der bekannten zwei Federn, die häufig aus Metall sind. Außerdem hat sich gezeigt, dass Exoskelette mit der vorgeschlagenen Vorrichtung besonders einfach in der Handhabung sind. Beide Vorteile erhöhen die Akzeptanz von Exoskeletten durch die Arbeiter und Anwender und tragen auf diese Weise dazu bei, Gesundheitsrisiken für den Arbeiter und Exoskelett-Nutzer zu reduzieren und ihm, trotz körperlich belastender Arbeit, eine lange Beschwerdefreiheit zu ermöglichen.

Es ist im Sinne der Erfindung bevorzugt, dass das Kraftübertragungselement von einem Seil gebildet wird. Das Kraftübertragungselement weist ein erstes und ein zweites Ende auf, wobei die beiden Enden des Kraftübertragungselements vorzugsweise auch als Seilenden bezeichnet werden können, wenn das Kraftübertragungselement der Vorrichtung von einem Seil gebildet wird. Das Kraftübertragungselement kann aus natürlichen Fasern oder Kunststoff hergestellt sein und ist insbesondere dazu eingerichtet, Zugbelastungen sicher auszuhalten, ohne zu reißen. Es ist im Sinne der Erfindung bevorzugt, dass das Kraftübertragungselement im Wesentlichen starr ausgebildet ist, während das Federelement im Wesentlichen elastisch ausgebildet ist. Vorzugsweise unterscheiden sich die elastischen Eigenschaften des Kraftübertragungselements und des Federelements in diesem Sinne deutlich voneinander. Es ist im Sinne der Erfindung insbesondere bevorzugt, dass das Federelement einen Großteil bzw. den größeren Teil der elastischen Verformung innerhalb der vorgeschlagenen Vorrichtung bewirkt, während die elastische Verformung, die durch das Kraftübertragungselement hervorgerufen wird, vemachlässigbar klein ist.

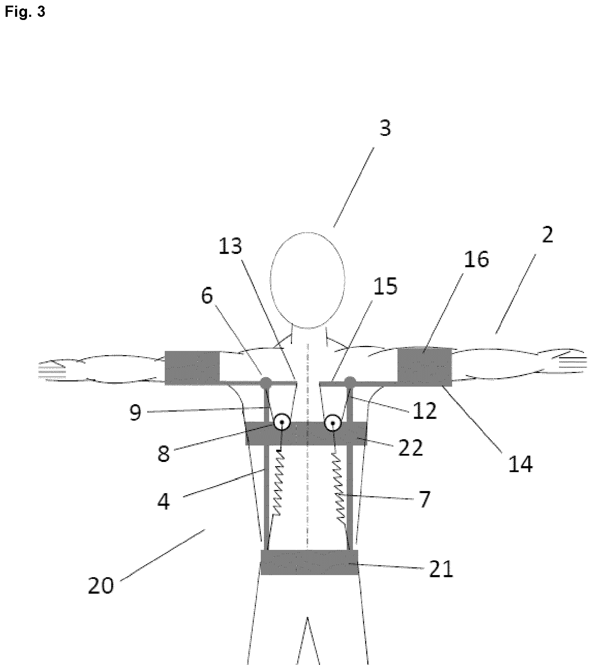

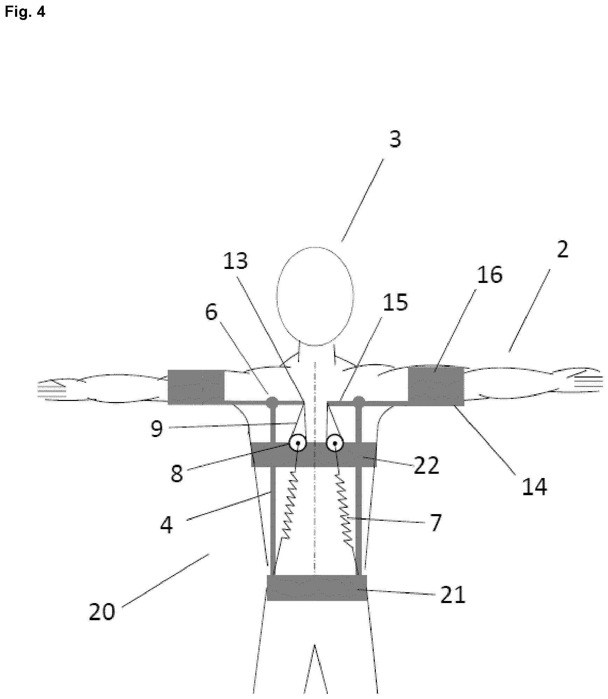

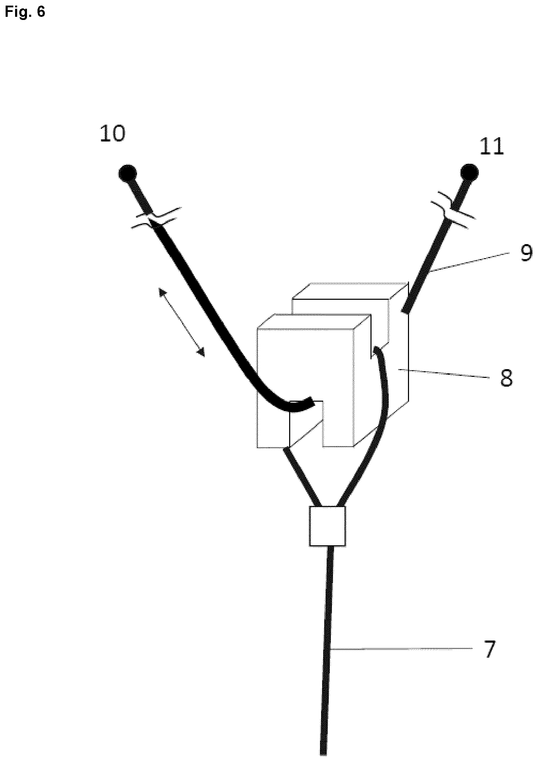

Es ist im Sinne der Erfindung bevorzugt, dass das Verbindungselement von einer Umlenkrolle gebildet wird. Sie wird im Sinne der Erfindung bevorzugt auch als Rolle bezeichnet und dient zur Führung eines Seils o.Ä. Eine Rolle stellt im Sinne der Erfindung bevorzugt eine mit einer Rille versehene Scheibe zum Umlenken einer seilartigen Vorrichtung dar. Sie kann als Kraftwandler eingesetzt werden und verbindet im Kontext der vorliegenden Erfindung das Federelement mit dem Kraftübertragungselement. Ein entsprechendes Ausführungsbeispiel der Erfindung, bei dem das Verbindungselement von einer Umlenkrolle gebildet wird, ist in Fig. 6 dargestellt. In dieser Ausführungsform der Erfindung ist es bevorzugt, dass das Kraftübertragungselement, in der Rille der Rolle geführt wird, wobei die Rolle den tiefsten Punkt in Bezug auf die Vorrichtung markiert, den das Kraftübertragungselement einnehmen kann. Der Begriff "tiefster Punkt" ist für den Fachmann nicht unklar, weil der Fachmann weiß, dass im Kontext der vorliegenden Erfindung die in Richtung des Erdmittelpunkts wirkende Gravitationskraft die Raumrichtung "nach unten" definiert. Demgemäß sind Objekte, die einen geringeren Abstand zum Erdboden aufweisen, unterhalb von solchen anderen Objekten angeordnet, die einen größeren Abstand zum Erdboden aufweisen. Die Formulierung, dass die Umlenkrolle den tiefsten Punkt in Bezug auf die Vorrichtung markiert, den das Kraftübertragungselement einnehmen kann, bedeutet somit im Sinne der Erfindung bevorzugt, dass der überwiegende Teil des Kraftübertragungselements oberhalb des Verbindungselements angeordnet vorliegt. Vorzugsweise liegt das Verbindungselement im Wesentlichen mittig in Bezug auf das Kraftübertragungselement vor oder teilt mit anderen Worten das Kraftübertragungselement in zwei Hälften. Die beiden Hälften des Kraftübertragungselements können eine Ebene aufspannen, die im Wesentlichen parallel zu einer Rückenebene des Nutzers liegt, insbesondere wenn die Arme des Nutzers seitlich ausgetreckt sind, wie beispielsweise in den Fig. 3 und 4 dargestellt. Vorzugsweise liegt auch das Federelement in dieser Rückenebene der Vorrichtung vor.

Es kann im Sinne der Erfindung auch bevorzugt sein, dass das Verbindungselement von einem Verbindungselement gebildet wird, das beispielsweise einen im Wesentlichen quaderförmigen Grundkörper aufweist. Ein entsprechendes Beispiel ist in Fig. 6 dargestellt. Das Verbindungselement in dieser bevorzugten Ausgestaltung der Erfindung kann vorzugsweise als quaderförmiges Verbindungselement bezeichnet werden. Es weist vorzugsweise zwei Aussparungen auf, wobei eine obere Aussparung der Befestigung des Federelements dient. Beispielsweise kann eine Schlaufe, die den oberen Abschluss des Federelements bildet, in die obere Aussparung des quaderförmigen Verbindungselements eingehängt werden. Eine untere Aussparung des Verbindungselements kann vorzugsweise für die Aufnahme des Kraftübertragungselements verwendet werden. Insbesondere kann die untere Aussparung des Verbindungselements eine ähnliche Funktion bereitstellen, wie die Rille einer Umlenkrolle, in der das Kraftübertragungselement geführt werden kann. Es ist im Sinne der Erfindung bevorzugt, dass die obere Aussparung durch eine nach oben weisende Öffnung gekennzeichnet wird, während die untere Aussparung des Verbindungselements durch eine in die Raumrichtung nach unten weisende Öffnung gekennzeichnet wird. Vorzugsweise teilt auch das quaderförmige Verbindungselement bzw. seine untere Aussparung das Kraftübertragungselement in zwei Hälften. Die Hälften können auch in dieser Ausführungsform der Erfindung eine Ebene aufspannen, wobei die von den Hälften des Kraftübertragungselements aufgespannte Ebene im Wesentlichen orthogonal zu einer Rückenebene des Nutzers angeordnet sein kann und von der Lage der Aufhängepunkte bestimmt wird. Es ist im Sinne der Erfindung bevorzugt, dass das Verbindungselement dazu eingerichtet ist, eine Kraft des Federelements in beispielsweise zwei Kraftstränge aufzuteilen. Dabei wirkt ein erster Kraftstrang vorzugsweise zwischen dem Verbindungselement und die ersten Aufhängepunkt und ein zweiter Kraftstrang vorzugsweise zwischen dem Verbindungselement und dem zweiten Aufhängepunkt. Wenn durch das Verbindungselement eine Aufteilung der Kraft des Federelements in zwei Kraftstränge erfolgt, ist es im Sinne der Erfindung bevorzugt, dass der erste Kraftstrang und der zweite Kraftstrang im Wesentlichen gleich groß sind. Dadurch liegt an beiden Aufhängepunkten vorzugsweise jeweils eine im Wesentlichen gleicht große Kraft an, wodurch eine besonders gleichmäßige und rückenschonende Bereitstellung der Unterstützungskraft für den Nutzer bzw. seine Arme gewährleistet wird.

Die Stützvorrichtung kann im Sinne der Erfindung auch als Stützstruktur bezeichnet werden. Sie kann vorzugsweise als Strebe oder als Stange ausgebildet sein und liegt insbesondere zwischen dem Hüftgurt eines Exoskeletts, das mindestens eine vorgeschlagene Vorrichtung umfasst, und einer Armstrebe der Vorrichtung angeordnet vor. An ihrem unteren Ende kann die Stützvorrichtung vorzugsweise mit einem Kugelgelenk in den Hüftgurt einmünden. In ihrem oberen Bereich wird die Verbindung zu der Armstrebe vorzugsweise über das Drehgelenk hergestellt.

In einer bevorzugten Ausführungsform der Erfindung kann die Vorrichtung insbesondere ein Federelement zur Bereitstellung der Unterstützungskraft umfassen, wobei das Federelement zwischen der Stützstruktur und einer Umlenkrolle angeordnet vorliegt, wobei die Vorrichtung ein Seil umfasst, das um die Rolle führbar ist und dessen Enden an einem ersten Aufhängepunkt an der Stützvorrichtung und einem zweiten Aufhängepunkt an der Armstrebe anbringbar sind, wobei im ersten Modus ein erstes Seilende am ersten Aufhängepunkt und ein zweites Seilende am zweiten Aufhängepunkt angebracht vorliegt und im zweiten Modus das erste und das zweite Seilende am zweiten Aufhängepunkt angebracht vorliegen. In dieser bevorzugten Ausführungsform der Erfindung kann es bevorzugt sein, dass im ersten Modus ein erstes Seilende am ersten Aufhängepunkt und ein zweites Seilende am zweiten Aufhängepunkt angebracht vorliegt und im zweiten Modus das erste und das zweite Seilende am zweiten Aufhängepunkt angebracht vorliegen.

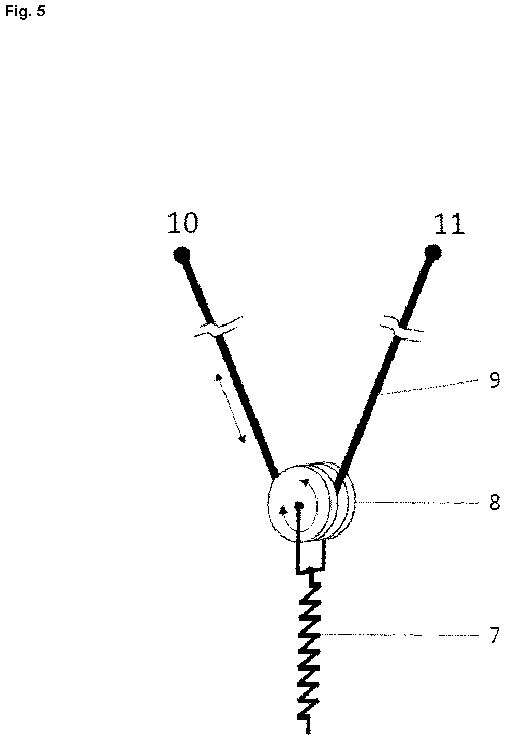

Es ist im Sinne der Erfindung bevorzugt, dass das eine Federelement der Vorrichtung von einem Expander gebildet wird. Ein Expander stellt im Sinne der Erfindung bevorzugt eine seilartige Vorrichtung dar, die über elastische Eigenschaften verfügt. Beispielsweise kann das Federelement ein elastisches Band, eine Spannschnur, ein Gummiband, ein Gummiseil oder eine Gummischnur umfassen, ohne darauf beschränkt zu sein. Das Federelement kann in einer alternativen Ausführungsform auch von einer Feder, wie einer Schraubenfeder, gebildet werden. Das Federelement liegt erfindungsgemäß zwischen der Stützstruktur und einem Verbindungselement angeordnet vor. Es ist im Sinne der Erfindung bevorzugt, dass das Federelement ein unteres Ende und ein oberes Ende aufweist, wobei das untere Ende an der Stützstruktur befestigt werden kann und das obere Ende des Federelements im Bereich des Verbindungselements endet. Beispielsweise kann das Federelement an seinem oberen Ende eine Schlaufe bilden, wobei die Schlaufe des Federelements von unten um das Verbindungselement herumgeführt werden kann. Diese Ausgestaltung des Federelements ist insbesondere dann bevorzugt, wenn das Federelement von einer seilartigen Vorrichtung, wie einem Expander oder einem elastischen Seil gebildet wird. Ein entsprechendes Beispiel wird in Fig. 6 dargestellt. Es kann im Sinne der Erfindung auch bevorzugt sein, dass das Federelement mit einem zusätzlichen Befestigungsmittel, wie einer Öse, einem Haken oder einem Bügel an dem Verbindungselement befestigt wird. Diese Ausgestaltung des Federelements ist insbesondere dann bevorzugt, wenn das Federelement von einer Feder, beispielsweise einer Schraubenfeder, gebildet wird. Ein entsprechendes Beispiel wird in Fig. 5 dargestellt.

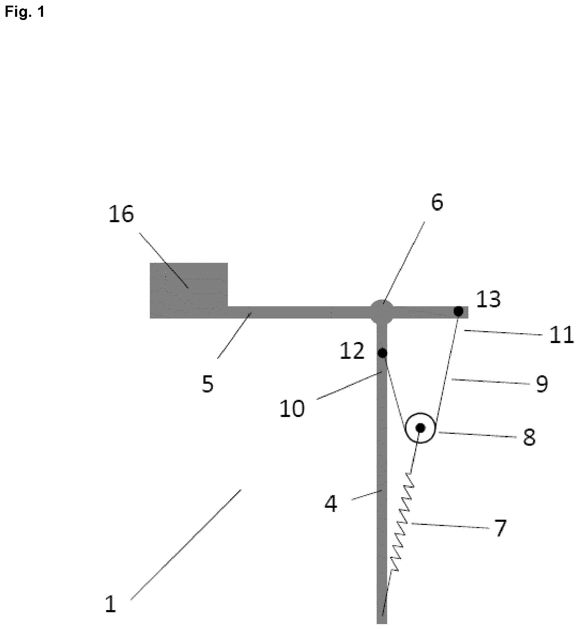

Es ist gemäß der vorliegenden Erfindung vorgesehen, dass das Kraftübertragungselement zwei Enden aufweist, wobei die Enden an einem ersten Aufhängepunkt und einem zweiten Aufhängepunkt angebracht werden können. Der erste Aufhängepunkt liegt vorzugsweise im Bereich der Stützstruktur vor. Es ist im Sinne der Erfindung besonders bevorzugt, dass der erste Aufhängepunkt in einem oberen Bereich der Stützstruktur vorliegt, wobei der obere Bereich der Stützstruktur in der Nähe des Drehgelenks liegt, das die Stützstruktur mit der Armstrebe verbindet. Es ist erfindungsgemäß vorgesehen, dass die Vorrichtung eine Stützvorrichtung und eine Armstrebe umfasst, die über das Drehgelenk derart miteinander verbunden vorliegen, dass die Armstrebe in Bezug auf die Stützvorrichtung um eine Rotationsachse drehbar gelagert ist. Vorzugsweise liegt der erste Aufhängepunkt unterhalb dieses Drehgelenks vor. Eine mögliche Anordnung des ersten Aufhängepunkts ist in Fig. 1 dargestellt. Der zweite Aufhängepunkt der Vorrichtung liegt vorzugsweise im Bereich der Armstrebe vor. Es ist im Sinne der Erfindung besonders bevorzugt, dass der zweite Aufhängepunkt an einem inneren Ende der Armstrebe angeordnet vorliegt. Vorzugsweise unterteilt das Drehgelenk die Armstrebe in einen längeren äußeren Bereich und einen kürzeren inneren Bereich.

Der äußere Bereich der Armstrebe liegt vorzugsweise in einem äußeren Bereich des Rumpfes oder des Rückens des Nutzers vor, wobei dieser äußere Bereich insbesondere der Wirbelsäule des Nutzers abgewandt ist. Der äußere Bereich der Armstrebe kann vorzugsweise auch als distaler Bereich der Armstrebe bezeichnet werden. Vorzugsweise weist der distale Bereich der Armstrebe eine größere Länge auf als der innere Bereich der Armstrebe, der im Sinne der Erfindung vorzugsweise auch als proximaler Bereich der Armstrebe bezeichnet werden kann. Der innere oder proximale Bereich der Armstrebe ist vorzugsweise der Wirbelsäule des Nutzers zugewandt, wobei der zweite Aufhängepunkt in einer besonders bevorzugten Ausführungsform der Erfindung am inneren Ende der Armstrebe vorliegen kann. Mit andere Worten liegt der zweite Aufhängepunkt in unmittelbarer Nähe zur Wirbelsäule des Nutzers vor.

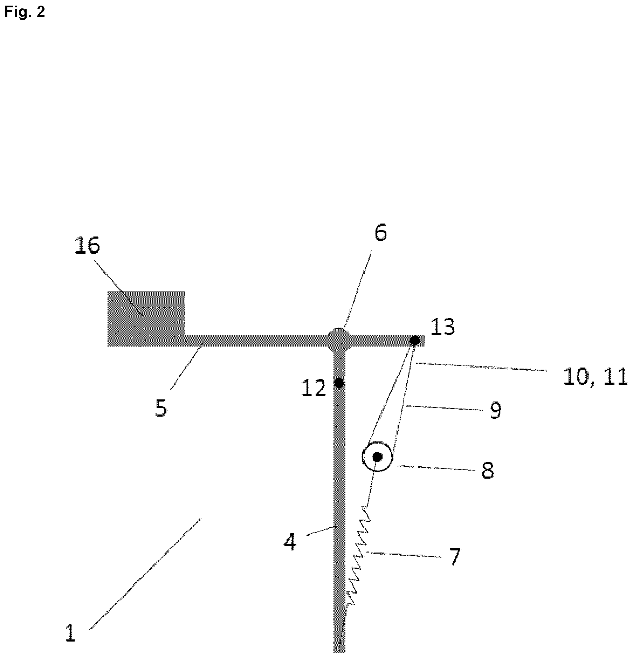

Ein besonderer Vorteil der vorgeschlagenen Erfindung besteht darin, dass mit der vorliegenden Erfindung ein vorzugsweise passives Exoskelett bereitgestellt werden kann, das in zwei Betriebsmodi betrieben bzw. genutzt werden kann. Diese Betriebszustände werden im Sinne der Erfindung bevorzugt als erster und zweiter Modus bezeichnet. Es ist im Sinne der Erfindung bevorzugt, dass der erste Modus einem ersten Kraftbereich entspricht und der zweite Modus einem zweiten Kraftbereich, wobei die der Nutzer des Exoskeletts in den unterschiedlichen Modi unterschiedlich große Unterstützungskräfte, beispielsweise bei der Durchführung von Überkopfarbeiten, erfährt. Es ist im Sinne der Erfindung besonders bevorzugt, dass dem Nutzer mit der vorliegenden Erfindung je nach eingestelltem Modus unterschiedlich große Unterstützungskräfte bei der Durchführung von Überkopfarbeiten zur Verfügung gestellt werden können. Die Bereitstellung der unterschiedlich großen Unterstützungskräfte wird insbesondere durch die unterschiedliche Aufhängung der Kraftübertragungselement-Enden an dem ersten und/oder dem zweiten Aufhängepunkt ermöglicht. Erfindungsgemäß ist vorgesehen, dass im ersten Modus ein erstes Ende des Kraftübertragungselements am ersten Aufhängepunkt und ein zweites Ende des Kraftübertragungselements am zweiten Aufhängepunkt angebracht vorliegt. Diese Anordnung des Kraftübertragungselements wird beispielsweise in den Fig. 1 und 3 dargestellt. In diesem ersten Modus wird dem Nutzer durch die spezifische Aufhängung des Kraftübertragungselements eine kleinere Unterstützungskraft zur Verfügung gestellt als im zweiten Modus. Im zweiten Modus oder im zweiten Betriebszustand der Vorrichtung bzw. des Exoskeletts liegen das erste und das zweite Ende des Kraftübertragungselements am zweiten Aufhängepunkt angebracht vor. Die spezifische Anordnung des Kraftübertragungselements gemäß dem zweiten Betriebs- oder Kraftmodus wird beispielsweise in den Fig. 2 und 4 dargestellt und ermöglicht vorteilhafterweise eine besonders große Unterstützungskraft für den Nutzer.

Es ist im Sinne der Erfindung bevorzugt, dass der erste Modus einem ersten Kraftbereich entspricht und der zweite Modus einem zweiten Kraftbereich. Beispielsweise kann dem Arbeiter im ersten Modus eine kleinere Unterstützungskraft für die oberen Extremitäten bereitgestellt werden als im zweiten Modus. Die Aufhängung im ersten Modus kann vorzugsweise auch dadurch beschrieben werden, dass die Aufhängung des Kraftübertragungselements am ersten und am zweiten Aufhängepunkt erfolgt. Im zweiten Modus werden beide Enden des Kraftübertragungselements vorzugsweise am selben Aufhängepunkt, insbesondere dem zweiten Aufhängepunkt, befestigt. Dadurch kann vorteilhafterweise eine größere Unterstützungskraft erzeugt werden als im ersten Modus oder Betriebszustand der Vorrichtung.

Die Vorrichtung umfasst eine Stützvorrichtung und eine Armstrebe, wobei die Stützvorrichtung vorzugsweise im Wesentlichen parallel zu einer gedanklichen Achse angeordnet ist, wobei die Achse im Wesentlichen parallel zur Wirbelsäule des Nutzers verläuft. Der Begriff "im Wesentlichen" erzeugt für den Fachmann keine Unklarheit, weil der Fachmann weiß, dass beim Tragen der Vorrichtung bzw. des Exoskeletts eine Abweichung von der exakten mathematischen Parallelität auftreten kann, die aber im Sinne der Erfindung immer noch unter die Formulierung "im Wesentlichen parallel" fallen soll. Es ist im Kontext der vorliegenden Erfindung bevorzugt, dass die Stützstruktur ein unteres und ein oberes Ende aufweist, wobei das das obere Ende in dem Drehgelenk mündet. Das untere Ende kann vorzugsweise in einem Rumpfbereich des Nutzers der Vorrichtung enden. Die vorgeschlagene Vorrichtung kann vorzugsweise in einem Exoskelett verwendet werden, wobei das Exoskelett vorzugsweise einen Hüftgurt umfassen kann, in den die Stützstruktur einmündet. Die Einmündung der Stützstruktur in den Hüftgurt bzw. die Verbindung zwischen Hüftgurt und Stützstruktur kann beispielsweise ein Gelenk umfassen, das beispielsweise als Kugelgelenk ausgebildet sein kann. Die Verwendung von Kugelgelenken an dieser Stelle eines Exoskeletts hat sich als vorteilhaft herausgestellt, weil dem Nutzer dadurch eine Beweglichkeit bzw. eine Bewegungsfreiheit in möglichst viele Richtungen, d.h. vorzugsweise mit vielen Freiheitsgraden, ermöglicht wird.

Es ist erfindungsgemäß vorgesehen, dass die Vorrichtung eine Stützvorrichtung und eine Armstrebe umfasst, die über das Drehgelenk derart miteinander verbunden vorliegen, dass die Armstrebe in Bezug auf die Stützvorrichtung um eine Rotationsachse drehbar gelagert ist. Es ist im Sinne der Erfindung bevorzugt, dass die Rotationsachse des Drehgelenks im Wesentlichen orthogonal zu der gedanklichen Achse angeordnet ist, die im Wesentlichen parallel zur Wirbelsäule des Nutzers verläuft. Die Rotationsachse ragt vorzugsweise aus einer Rückenebene des Nutzers heraus, wobei die Rückenebene im Wesentlichen durch die Rückenfläche des Nutzers ausgespannt wird. Vorzugsweise ist die Rotationsachse des Drehgelenks im Wesentlichen orthogonal zur Rückenebene des Nutzers ausgebildet bzw. die Rotationsachse steht im Wesentlichen senkrecht auf der Rückenebene des Nutzers. Dadurch ist die Armstrebe vorteilhafterweise in Bezug auf die Stützstruktur drehbar ausgebildet, wobei die Drehbewegung der Armstrebe insbesondere in der Rückenebene erfolgen kann.

Es ist im Sinne der Erfindung bevorzugt, dass das Federelement dazu eingerichtet ist, ein Drehmoment um die Rotationsachse herum zu erzeugen. Es ist im Sinne der Erfindung bevorzugt, dass das Federelement für eine Unterstützungskraft für den Nutzer sorgt, wobei die Unterstützungskraft vorzugsweise an der Armstrebe angreift. Dadurch wird vorteilhafterweise ein Drehmoment um die Rotationsachse erzeugt. Vorzugsweise ist die Armstrebe bezüglich der Stützstruktur um die Rotationsachse drehbar gelagert.

Es ist im Sinne der Erfindung bevorzugt, dass die Armstrebe eine Aufnahmevorrichtung für eine obere Extremität aufweist, wobei die Aufnahmevorrichtung vorzugsweise als Armschale ausgebildet sein kann. Vorzugsweise liegt die Aufnahmevorrichtung in dem äußeren Bereich der Armstrebe angeordnet vor, d.h. sie weist einen großen Abstand zur Wirbelsäule des Nutzers auf. Durch diesen großen Abstand der Aufnahmevorrichtung zur Wirbelsäule wird die Unterstützungswirkung der vorgeschlagenen Vorrichtung vom Nutzer als besonders entlastend und das Tragen der Vorrichtung als besonders komfortabel empfunden. Eine beispielhafte Anordnung der Armschale ist beispielsweise in den Fig. 1 bis 4 dargestellt. Die Aufnahmevorrichtung ist insbesondere dazu eingerichtet, die oberen Extremitäten des Nutzers der Vorrichtung bzw. des Exoskeletts aufzunehmen. Die oberen Extremitäten des Nutzers sind vorzugsweise die Arme des Nutzers. Die Vorrichtung und das vorgeschlagene Exoskelett werden vorzugsweise so von einem Nutzer am Körper getragen, dass insbesondere die Oberarme von der Aufnahmevorrichtung aufgenommen werden. Es ist im Sinne der Erfindung insbesondere bevorzugt, dass ein unterer Bereich der Oberarme in der Armschale der Armstrebe zu liegen kommt. Der untere Bereich des Oberarms ist vorzugsweise derjenige Bereich des Oberarms, der vom Ellenbogen ausgeht bzw. direkt oberhalb des Ellenbogens angeordnet vorliegt.

In einem zweiten Aspekt betrifft die Erfindung ein Exoskelett zur Unterstützung der oberen Extremitäten eines Nutzers. Das Exoskelett ist dadurch gekennzeichnet, dass es eine oder mehrere der vorgeschlagenen Vorrichtungen umfasst. Schematische Abbildungen von beispielhaften Ausführungsformen des Exoskeletts sind in den Fig. 3 und 4 dargestellt. Die Definitionen, technischen Wirkungen und Vorteile, die für die Vorrichtung beschrieben werden, gelten für das Exoskelett analog, und umgekehrt. Vorzugsweise handelt es sich bei dem vorgeschlagenen Exoskelett um ein passives Exoskelett, das insbesondere keine aktiven Komponenten, wie Motoren oder Antriebsmittel, umfasst. Es ist im Sinne der Erfindung bevorzugt, dass die Vorrichtungen im Kontext des vorgeschlagenen Exoskeletts im Wesentlichen symmetrisch zu einer Sagittalebene angeordnet sind, dies insbesondere, wenn das Exoskelett zwei Vorrichtungen umfasst. Das bedeutet im Sinne der Erfindung bevorzugt, dass die Vorrichtungen achsensymmetrisch um eine Achse, die im Wesentlichen parallel zur Wirbelsäule verläuft, angeordnet sind. Die Vorrichtungen können mit verschiedenen Befestigungsmitteln, wie Gurten, Geschirren, Bändern, Schnallen oder dergleichen aneinander und an einem Nutzer befestigt werden. Beispielsweise kann das Exoskelett einen Hüftgurt umfassen, an dem die unteren Enden der Stützvorrichtungen angeordnet vorliegen. Es ist im Sinne der Erfindung bevorzugt, dass die Stützvorrichtungen mittels Kugelgelenken an dem Hüftgurt befestigbar sind. Die Bereiche des Hüftgurtes, in die die Stützvorrichtungen einmünden, können vorzugsweise als Verbindungsbereiche bezeichnet werden. Vorzugsweise erfolgt in diesen Verbindungsbereichen auch die Verbindung zwischen dem Hüftgurt und den Federelementen der vorgeschlagenen Vorrichtung. Diese liegen vorzugsweise an einem unteren Bereich der Stützvorrichtung befestigt vor, wobei dieser untere Bereich der Stützvorrichtung zumindest teilweise mit dem Verbindungsbereich zwischen Hüftgurt und den Stützvorrichtungen zusammenfallen kann. Vorzugsweise umfasst der Verbindungsbereich die Kugelgelenke, mit denen die Stützvorrichtungen an dem Hüftgurt befestigt werden können. Die Verwendung von Kugelgelenken ermöglicht eine besonders große Beweglichkeit für den Nutzer, so dass er durch die Vorrichtung bzw. das Exoskelett möglichst wenig in seiner Bewegungsfreiheit eingeschränkt wird. Das Exoskelett kann beispielsweise auch ein Mittel zur Befestigung des Exoskeletts oder der Vorrichtung(an) am Oberkörper umfassen. Dabei kann es sich beispielsweise um einen Brustgurt handeln, der zum Beispiel auf Höhe der Verbindungselemente bzw. ein wenig unterhalb der Verbindungselemente angeordnet sein kann. Eine mögliche Ausführungsform des Exoskeletts mit einem Brustband ist beispielsweise in den Fig. 3 und 4 dargestellt.

Vorzugsweise wird durch die Verbindung von zwei Vorrichtungen ein Exoskelett erhalten, mit dem eine Unterstützungskraft für beide Oberarme des Nutzers bereitgestellt werden kann. Es sind jedoch auch Exoskelette vorstellbar, die beispielsweise nur eine Vorrichtung aufweisen. Dies kann in einigen Anwendungsfällen sinnvoll sein, wenn bei Überkopfarbeiten nur ein Arm zur Ausführung der Arbeiten verwendet wird oder wenn der Nutzer nur einen Arm verwenden kann, weil der zweite Arm beispielsweise verletzt ist oder aufgrund einer Verletzung oder eines Unfalls fehlt.

Die Erfindung betrifft in einem Ausführungsbeispiel, das im Folgenden beschrieben wird, insbesondere ein passives Exoskelett für die oberen Extremitäten, wobei das Exoskelett eine Unterstützungskraft bereitstellt, um zumindest einen Teil der Gravitationskräfte zu kompensieren. Die Vorrichtung bzw. das Exoskelett besteht aus einer Armstrebe, die über eine Armschale mit dem Oberarm des Nutzers verbunden wird und einer Stützstruktur, die mit dem Rumpf und/oder Oberkörper des Nutzers verbunden wird. Die Armstrebe ist bezüglich der Stützstruktur um eine Rotationsachse drehbar gelagert. Zwischen der Armstrebe und der Stützstruktur sorgt ein Federelement für eine Unterstützungskraft, die an der Armstrebe angreift und damit ein Drehmoment um die Rotationsachse erzeugt. Das Federelement ist an einem Ende mit der Stützstruktur und an einem anderen Ende mit einer Umlenkrolle verbunden, die als Verbindungselement fungiert. Um die Rolle wird ein Seil als Kraftübertragungselement geschlungen, wobei die Enden des Seils mit einem ersten und einem zweiten Aufhängpunkt verbunden werden können. Die Erfindung besitzt vorteilhafterweise zwei verschiedene Betriebszustände, die unterschiedliche Unterstützungskräfte aufweisen. In Betriebszustand 1 (erster Modus) ist ein Seilende mit der Armstrebe (zweiter Aufhängpunkt) verbunden und das andere Seilende mit der Stützstruktur (erster Aufhängpunkt) verbunden. In Betriebszustand 2 (zweiter Modus) sind beide Seilenden mit dem zweiten Aufhängpunkt an der Armstrebe verbunden, wodurch eine größere Unterstützungskraft ermöglicht wird.

Weitere Vorteile der Erfindung ergeben sich aus der folgenden Figurenbeschreibung. Die Figuren, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

In den Figuren sind gleiche und gleichartige Komponenten mit gleichen Bezugszeichen beziffert. Es zeigen:
- Fig. 1: schematische Darstellung einer bevorzugten Ausgestaltung der Vorrichtung im ersten Modus
- Fig. 2: schematische Darstellung einer bevorzugten Ausgestaltung der Vorrichtung im zweiten Modus
- Fig. 3: Darstellung einer bevorzugten Ausgestaltung des Exoskeletts im ersten Modus
- Fig. 4: Darstellung einer bevorzugten Ausgestaltung des Exoskeletts im zweiten Modus
- Fig. 5: verschiedene bevorzugte Ausgestaltungen des Verbindungselements und des Federelements
- Fig. 6: verschiedene bevorzugte Ausgestaltungen des Verbindungselements und des Federelements

### Ausführungsbeispiele der Erfindung:

Figur 1 zeigt eine schematische Darstellung einer bevorzugten Ausgestaltung der Vorrichtung (1) im ersten Modus, in dem vorzugsweise eine kleinere Unterstützungskraft für den Nutzer (3) bereitgestellt wird als im zweiten Modus. Dargestellt ist in Fig. 1 die Armstrebe (5), die über ein Drehgelenk (6) mit einer Stützvorrichtung (4) bzw. einer Stützstruktur (4) verbunden ist. In Bezug auf die Stützvorrichtung (4) ist die Armstrebe (5) drehbar gelagert, und zwar um eine Dreh- oder Rotationsachse, die durch das Drehgelenk (6) führt. Die Stützvorrichtung (4) verläuft vorzugsweise im Wesentlichen parallel zur Wirbelsäule des Nutzers (3). Von der Stützvorrichtung (4) geht ein Federelement (7) ab, dessen oberes Ende an einem Verbindungsmittel (8) befestigt vorliegt. Mit anderen Worten liegt das Federelement (7) zwischen der Stützstruktur (4) und dem Verbindungsmittel (8) angeordnet vor. Das Verbindungsmittel (8) kann beispielsweise als Umlenkrolle ausgebildet sein. Es ist vorzugsweise dazu eingerichtet, dass ein Kraftübertragungselement (9) um das Verbindungsmittel (8) herumgeführt werden kann. Dazu weist das Verbindungsmittel (8) vorzugsweise eine Rille oder eine Nut auf. Das Kraftübertragungsmittel (9) kann beispielsweise als Seil ausgebildet sein. Es weist zwei Enden (10, 11) auf, wobei die beiden Enden (10, 11) des Kraftübertragungselements (9) an zwei Aufhängepunkten (12, 13) angebracht werden können. Der erste Aufhängungspunkt (12) ist vorzugsweise an der Stützvorrichtung (4) angeordnet, während der zweite Aufhängepunkt (13) an einem innersten Ende der Armstrebe (5) angeordnet vorliegt. Das Drehgelenk (6) unterteilt die Armstrebe (5) vorzugsweise in einen äußeren Bereich (14) und einen inneren Bereich (15). Es ist im Sinne der Erfindung bevorzugt, dass der äußere Bereich (14) eine größere Länge aufweist als der innere Bereich (15) der Vorrichtung (1). Vorzugsweise liegt der zweite Aufhängepunkt (13) am äußersten Ende des inneren Bereichs (15) der Armstrebe (5) vor, also besonders nahe an der Wirbelsäule des Nutzers (3). In dem in Fig. 1 dargestellten Ausführungsbeispiel liegt die Vorrichtung (1) im ersten Modus vor, in dem das erste Ende (10) des Kraftübertragungsmittels (9) am ersten Aufhängepunkt (12) befestigt vorliegt, während das zweite Ende (11) des Kraftübertragungsmittels (9) am zweiten Aufhängepunkt (13) befestigt vorliegt. Durch diese Anordnung wird eine kleine Unterstützungskraft auf die oberen Extremitäten (2) des Nutzers übertragen (3). Die Armstrebe (5) der Vorrichtung (1) kann eine Aufnahmevorrichtung (16) umfassen, die beispielsweise als Armschale ausgebildet sein kann. Der Nutzer (3) kann dort beispielsweise seinen Oberarm (2) ablegen, um bequem über Kopf arbeiten zu können.

Fig. 2 zeigt eine schematische Darstellung einer bevorzugten Ausgestaltung der Vorrichtung (1) im zweiten Modus. Anders als in Fig. 1 sind im zweiten Modus oder Betriebszustand der Vorrichtung (1) beide Enden (10, 11) des Kraftübertragungselements (9) am zweiten Aufhängepunkt (13) der Vorrichtung befestigt, wobei der zweite Aufhängepunkt (13) vorzugsweise Bestandteil der Armstrebe (5) ist. Dadurch kann vorteilhafterweise eine größere Unterstützungskraft für den Nutzer bereitgestellt werden als im ersten Modus der Vorrichtung (1).

Fig. 3 zeigt eine Darstellung einer bevorzugten Ausgestaltung des Exoskeletts (20) im ersten Modus. Das in Fig. 3 dargestellte Exoskelett (20) umfasst zwei vorgeschlagene Vorrichtungen (1), sowie einen Hüftgurt (21) und ein Brustband (22) oder einen Brustgurt. Der Hüftgurt (21) kann von einem Nutzer (3) um die Hüften gelegt und in bekannter Weise befestigt und geschlossen werden. Dadurch wird zumindest ein Teil des Gewichts des Exoskeletts (20) auf die Hüften des Nutzers (3) gelagert. Der Brustgurt (22) ist vorzugsweise in Höhe der Brust des Nutzers (3) angeordnet, wobei in dieser Höhe vorzugsweise auch die Verbindungsmittel (8) der Vorrichtungen (1) angeordnet sind. Die Stützvorrichtungen (4) gehen in dem in Fig. 3 dargestellten Zustand des Exoskeletts (20) im Wesentlichen senkrecht nach oben von dem Hüftgurt (21) ab, wobei die Stützvorrichtungen (4) insbesondere unter Verwendung von Kugelgelenken (nicht dargestellt) an dem Hüftgurt (21) befestigt vorliegen können. Vorzugsweise verlaufen die Stützstrukturen (4) in der in Fig. 3 dargestellten Ausgestaltung der Vorrichtung (1) im Wesentlichen parallel zu der gedanklichen Achse, die im Wesentlichen parallel zur Wirbelsäule des Nutzers verläuft. Die gedankliche Achse ist in den Fig. 3 und 4 durch die mittige gestrichelte Linie angedeutet. Zwischen je einem Verbindungsmittel (8) und dem unteren Bereich der Stützvorrichtungen (4) ist jeweils ein Federelement (7) angeordnet, das beispielsweise ein elastisches Band oder eine Feder, wie eine Spiral- oder eine Zugfeder, umfassen oder davon gebildet sein kann. Von oben kommend, wird um das Verbindungselement (8) herum das Kraftübertragungselement (9) geführt. In dem in Fig. 3 dargestellten ersten Modus des Exoskeletts (20) liegt das erste Ende (10) des Kraftübertragungsmittels (9) am ersten Aufhängepunkt (12) befestigt vorliegt, während das zweite Ende (11) des Kraftübertragungsmittels (9) am zweiten Aufhängepunkt (13) befestigt vorliegt. Vorzugsweise ist der erste Aufhängepunkt (12) Bestandteil der Stützvorrichtung (4), während der zweite Aufhängepunkt (13) an der Armstrebe (5) in unmittelbarer Nähe der Wirbelsäule des Nutzers (3) angeordnet vorliegt.

Fig. 4 zeigt eine Darstellung einer bevorzugten Ausgestaltung des Exoskeletts (20) im zweiten Modus. In dem in Fig. 4 dargestellten zweiten Modus des Exoskeletts (20) sind beide Enden (10, 11) des Kraftübertragungselements (9) am zweiten Aufhängepunkt (13) der Vorrichtung befestigt, wodurch vorteilhafterweise eine größere Unterstützungskraft für den Nutzer bereitgestellt werden kann als im ersten Modus der Vorrichtung (1).

Fig. 5 und 6 zeigen verschiedene bevorzugte Ausgestaltungen des Verbindungselements (8) und des Federelements (7). Insbesondere wird in den Figuren 5 und 6 das Kraftübertragungselement (9) gezeigt, sowie sein erstes Ende (10) und sein zweites Ende (11). In dem in Fig. 5 gezeigten Ausführungsbeispiel der Erfindung ist das Federelement (7) als Schraubenfeder ausgebildet, während das Verbindungselement (8) von einer Umlenkrolle gebildet wird. Das Kraftübertragungselement (9) wird von einem Seil gebildet. In dem in Fig. 6 gezeigten Ausführungsbeispiel der Erfindung wird das Federelement (7) von einem Expander gebildet, während das Verbindungselement (8) einen quaderförmigen Grundkörper umfasst. Das Kraftübertragungselement (9) wird von einem Seil gebildet. Es versteht sich, dass die verschiedenen Ausführungsformen der Erfindung, die in den Fig. 5 und 6 dargestellt sind, beliebig untereinander kombiniert werden können.

### Bezugszeichenliste

- 1: Vorrichtung
- 2: obere Extremität
- 3: Nutzer
- 4: Stützvorrichtung
- 5: Armstrebe
- 6: Drehgelenk
- 7: Federelement
- 8: Verbindungselement
- 9: Kraftübertragungselement
- 10: erstes Ende des Kraftübertragungselements
- 11: zweites Ende des Kraftübertragungselements
- 12: erster Aufhängepunkt
- 13: zweiter Aufhängepunkt
- 14: äußerer Bereich der Armstrebe
- 15: innerer Bereich der Armstrebe
- 16: Aufnahmevorrichtung
- 20: Exoskelett
- 21: Hüftgurt
- 22: Brustgurt

## Patentansprüche

1. Vorrichtung (1) zur Bereitstellung einer Unterstützungskraft für die oberen Extremitäten (2) eines Nutzers (3), wobei die Unterstützungskraft in einem ersten Modus und in einem zweiten Modus bereitgestellt wird, wobei die Vorrichtung (1) eine Stützvorrichtung (4) und eine Armstrebe (5) umfasst, die über ein Drehgelenk (6) derart miteinander verbunden vorliegen, dass die Armstrebe (5) in Bezug auf die Stützvorrichtung (4) um eine Rotationsachse drehbar gelagert ist,
wobei
die Vorrichtung (1) ein Federelement (7) zur Bereitstellung der Unterstützungskraft umfasst, wobei das Federelement (7) zwischen der Stützvorrichtung (4) und einem Verbindungselement (8) angeordnet vorliegt, wobei die Vorrichtung (1) ein Kraftübertragungselement (9) umfasst, das ein erstes Ende (10) und ein zweites Ende (11) aufweist und das um das Verbindungselement (8) führbar ist, wobei die Enden (10, 11) des Kraftübertragungselements (9) an einem ersten Aufhängepunkt (12) an der Stützvorrichtung (4) und einem zweiten Aufhängepunkt (13) an der Armstrebe (5) anbringbar sind, wobei im ersten Modus das erste Ende (10) des Kraftübertragungselements (9) am ersten Aufhängepunkt (12) und ein zweites Ende (11) des Kraftübertragungselements (9) am zweiten Aufhängepunkt (13) angebracht vorliegt und **dadurch gekennzeichnet, dass** im zweiten Modus das erste Ende (10) und das zweite Ende (11) des Kraftübertragungselements (9) am zweiten Aufhängepunkt (13) angebracht vorliegen.

2. Vorrichtung (1) nach Anspruch 1
**dadurch gekennzeichnet, dass**
die Stützvorrichtung (4) ein unteres und ein oberes Ende aufweist, wobei das obere Ende in dem Drehgelenk (6) mündet.

3. Vorrichtung (1) nach Anspruch 1 oder 2
**dadurch gekennzeichnet, dass**
das Federelement (7) dazu eingerichtet ist, ein Drehmoment um die Rotationsachse herum zu erzeugen.

4. Vorrichtung (1) nach einem der vorhergehenden Ansprüche
**dadurch gekennzeichnet, dass**
das Drehgelenk (6) die Armstrebe (5) in einen längeren äußeren Bereich (14) und einen kürzeren inneren Bereich (15) unterteilt.

5. Vorrichtung (1) nach einem der vorhergehenden Ansprüche
**dadurch gekennzeichnet, dass**
die Armstrebe (5) eine Aufnahmevorrichtung (16) für eine obere Extremität (2) aufweist

6. Vorrichtung (1) nach Anspruch 5
**dadurch gekennzeichnet, dass**
die Aufnahmevorrichtung (16) in dem äußeren Bereich (14) der Armstrebe (5) angeordnet vorliegt.

7. Vorrichtung (1) nach einem der vorhergehenden Ansprüche
**dadurch gekennzeichnet, dass**
der zweite Aufhängepunkt (13) an einem inneren Ende der Armstrebe (5) angeordnet vorliegt.

8. Vorrichtung (1) nach einem der vorhergehenden Ansprüche
**dadurch gekennzeichnet, dass**
die Rotationsachse des Drehgelenks (6) zwischen Armstrebe (5) und Stützvorrichtung (4) im Wesentlichen orthogonal zu einer gedanklichen Achse angeordnet ist, wobei die gedankliche Achse im Wesentlichen parallel zur Wirbelsäule des Nutzers (3) verläuft.

9. Vorrichtung (1) nach einem der vorhergehenden Ansprüche
**dadurch gekennzeichnet, dass**
das Verbindungselement dazu eingerichtet ist, eine Kraft des Federelements in zwei Kraftstränge aufzuteilen, wobei ein erster Kraftstrang zwischen dem Verbindungselement und die ersten Aufhängepunkt und ein zweiter Kraftstrang zwischen dem Verbindungselement und dem zweiten Aufhängepunkt wirkt.

10. Exoskelett (20) zur Unterstützung der oberen Extremitäten (2) eines Nutzers (3)
**dadurch gekennzeichnet, dass**
das Exoskelett (20) eine oder mehrere Vorrichtungen (1) nach einem der vorhergehenden Ansprüche umfasst.

11. Exoskelett (20) nach Anspruch 10
**dadurch gekennzeichnet, dass**
die Vorrichtungen (1) im Wesentlichen symmetrisch zu einer Sagittalebene des Nutzers (3) angeordnet sind.

12. Exoskelett (20) nach Anspruch 10 oder 11
**dadurch gekennzeichnet, dass**
das Exoskelett (20) einen Hüftgurt (21) umfasst, an dem die unteren Enden der Stützvorrichtungen (4) angeordnet vorliegen.

13. Exoskelett (20) nach Anspruch 12
**dadurch gekennzeichnet, dass**
die Stützvorrichtung (4) mittels einem Kugelgelenk an dem Hüftgurt (21) befestigbar ist.

14. Exoskelett (20) nach einem der Ansprüche Anspruch 10 bis 13
**dadurch gekennzeichnet, dass**
das Exoskelett (20) einen Brustgurt (22) zur Befestigung am Nutzer (3) umfasst.

## Claims

1. Device (1) for providing a supporting force for the upper extremities (2) of a user (3), wherein the supporting force is provided in a first mode and in a second mode, wherein the device (1) comprises a supporting device (4) and an arm strut (5), which are connected together via a rotary joint (6) such that the arm strut (5) is mounted so as to be rotatable about an axis of rotation with respect to the supporting device (4),
wherein
the device (1) comprises a spring element (7) for providing the supporting force, wherein the spring element (7) is arranged between the supporting device (4) and a connecting element (8), wherein the device (1) comprises a force transmission element (9) that has a first end (10) and a second end (11) and that is able to be guided about the connecting element (8), wherein the ends (10, 11) of the force transmission element (9) are able to be attached to the supporting device (4) at a first suspension point (12) and to the arm strut (5) at a second suspension point (13), wherein the first end (10) of the force transmission element (9) is attached to the first suspension point (12) and a second end (11) of the force transmission element (9) is attached to the second suspension point (13) in the first mode, and **characterized in that** the first end (10) and the second end (11) of the force transmission element (9) are attached to the second suspension point (13) in the second mode.

2. Device (1) according to Claim 1,
**characterized in that**
the supporting device (4) has a lower end and an upper end, wherein the upper end leads to the rotary joint (6).

3. Device (1) according to Claim 1 or 2,
**characterized in that**
the spring element (7) is designed to generate a torque about the axis of rotation.

4. Device (1) according to one of the preceding claims,
**characterized in that**
the rotary joint (6) divides the arm strut (5) into a longer outer region (14) and a shorter inner region (15).

5. Device (1) according to one of the preceding claims,
**characterized in that**
the arm strut (5) has a receiving device (16) for an upper extremity (2).

6. Device (1) according to Claim 5,
**characterized in that**
the receiving device (16) is arranged in the outer region (14) of the arm strut (5).

7. Device (1) according to one of the preceding claims,
**characterized in that**
the second suspension point (13) is arranged at an inner end of the arm strut (5).

8. Device (1) according to one of the preceding claims,
**characterized in that**
the axis of rotation of the rotary joint (6) between the arm strut (5) and supporting device (4) is arranged substantially orthogonally to an imaginary axis, wherein the imaginary axis extends substantially parallel to the spine of the user (3).

9. Device (1) according to one of the preceding claims,
**characterized in that**
the connecting element is designed to split a force of the spring element into two force paths, wherein a first force path acts between the connecting element and the first suspension point and a second force path acts between the connecting element and the second suspension point.

10. Exoskeleton (20) for supporting the upper extremities (2) of a user (3),
**characterized in that**
the exoskeleton (20) comprises one or more devices (1) according to one of the preceding claims.

11. Exoskeleton (20) according to Claim 10,
**characterized in that**
the devices (1) are arranged substantially symmetrically to a sagittal plane of the user (3).

12. Exoskeleton (20) according to Claim 10 or 11,
**characterized in that**
the exoskeleton (20) comprises a hip belt (21) at which the lower ends of the supporting devices (4) are arranged.

13. Exoskeleton (20) according to Claim 12,
**characterized in that**
the supporting device (4) is able to be fastened to the hip belt (21) by means of a ball joint.

14. Exoskeleton (20) according to one of Claims 10 to 13,
**characterized in that**
the exoskeleton (20) comprises a chest belt (22) for fastening to the user (3).

## Revendications

1. Dispositif (1) de fourniture d'une force de soutien pour les extrémités supérieures (2) d'un utilisateur (3), la force de soutien étant fournie dans un premier mode et dans un deuxième mode, le dispositif (1) comprenant un dispositif de support (4) et un étai pour bras (5), qui sont présents de manière reliée l'un à l'autre par le biais d'une articulation rotative (6) de telle sorte que l'étai pour bras (5) est monté rotatif autour d'un axe de rotation par rapport au dispositif de support (4),
le dispositif (1) comprenant un élément ressort (7) servant à la fourniture de la force de soutien, l'élément ressort (7) étant présent de manière disposée entre le dispositif de support (4) et un élément de liaison (8), le dispositif (1) comprenant un élément de transmission de force (9) qui présente une première extrémité (10) et une deuxième extrémité (11) et qui peut être guidé autour de l'élément de liaison (8), les extrémités (10, 11) de l'élément de transmission de force (9) pouvant être fixées sur le dispositif de support (4) au niveau d'un premier point de suspension (12) et sur l'étai pour bras (5) au niveau d'un deuxième point de suspension (13), la première extrémité (10) de l'élément de transmission de force (9) étant présente de manière fixée au niveau du premier point de suspension (12) et une deuxième extrémité (11) de l'élément de transmission de force (9) étant présente de manière fixée au niveau du deuxième point de suspension (13) dans le premier mode et **caractérisé en ce que**, dans le deuxième mode, la première extrémité (10) et la deuxième extrémité (11) de l'élément de transmission de force (9) sont présentes de manière fixée au niveau du deuxième point de suspension (13).

2. Dispositif (1) selon la revendication 1,
**caractérisé en ce que**
le dispositif de support (4) présente une extrémité inférieure et une extrémité supérieure, l'extrémité supérieure débouchant dans l'articulation rotative (6).

3. Dispositif (1) selon la revendication 1 ou 2,
**caractérisé en ce que**
l'élément ressort (7) est conçu pour produire un couple autour de l'axe de rotation.

4. Dispositif (1) selon l'une des revendications précédentes,
**caractérisé en ce que**
l'articulation rotative (6) divise l'étai pour bras (5) en une région extérieure plus longue (14) et une région intérieure plus courte (15).

5. Dispositif (1) selon l'une des revendications précédentes,
**caractérisé en ce que**
l'étai pour bras (5) présente un dispositif de réception (16) pour une extrémité supérieure (2).

6. Dispositif (1) selon la revendication 5,
**caractérisé en ce que**
le dispositif de réception (16) est présent de manière disposée dans la région extérieure (14) de l'étai pour bras (5).

7. Dispositif (1) selon l'une des revendications précédentes,
**caractérisé en ce que**
le deuxième point de suspension (13) est présent de manière disposée à une extrémité intérieure de l'étai pour bras (5).

8. Dispositif (1) selon l'une des revendications précédentes,
**caractérisé en ce que**
l'axe de rotation de l'articulation rotative (6) entre l'étai pour bras (5) et le dispositif de support (4) est disposé sensiblement orthogonalement à un axe imaginaire, l'axe imaginaire s'étendant sensiblement parallèlement à la colonne vertébrale de l'utilisateur (3).

9. Dispositif (1) selon l'une des revendications précédentes,
**caractérisé en ce que**
l'élément de liaison est conçu pour diviser une force de l'élément ressort en deux voies de force, une première voie de force agissant entre l'élément de liaison et le premier point de suspension et une deuxième voie de force agissant entre l'élément de liaison et le deuxième point de suspension.

10. Exosquelette (20) permettant de soutenir les extrémités supérieures (2) d'un utilisateur (3),
**caractérisé en ce que**
l'exosquelette (20) comprend un ou plusieurs dispositifs (1) selon l'une des revendications précédentes.

11. Exosquelette (20) selon la revendication 10,
**caractérisé en ce que**
les dispositifs (1) sont disposés sensiblement symétriquement par rapport à un plan sagittal de l'utilisateur (3).

12. Exosquelette (20) selon la revendication 10 ou 11,
**caractérisé en ce que**
l'exosquelette (20) comprend une ceinture sous-abdominale (21) au niveau de laquelle les extrémités inférieures des dispositifs de support (4) sont disposées.

13. Exosquelette (20) selon la revendication 12,
**caractérisé en ce que**
le dispositif de support (4) peut être fixé à la ceinture sous-abdominale (21) au moyen d'une articulation sphérique.

14. Exosquelette (20) selon l'une des revendications 10 à 13,
**caractérisé en ce que**
l'exosquelette (20) comprend une ceinture thoracique (22) servant à la fixation à l'utilisateur (3).
